# EUROPEAN PATENT SPECIFICATION

(11) **EP 2 768 567 B1**
(45) Date of publication and mention of the grant of the patent: **08.05.2019**
(21) Application number: 12788432.8
(22) Date of filing: 19.10.2012
(51) Int. Cl.: A61M 25/06

(54) **CATHETER WITH REMOVABLE CANNULA FOR PUNCTURING A BODY CAVITY AND CANNULA FOR THE USE WITH A CATHETER WHICH CAN BE MOVED IN THE CANNULA**
KATHETER MIT ABNEHMBARER KANÜLE ZUR PUNKTION EINES KÖRPERHOHLRAUMES UND KANÜLE ZUR VERWENDUNG MIT EINEM IN DER KANÜLE BEWEGLICHEN KATHETER
CATHÉTER AYANT UNE CANULE AMOVIBLE POUR PERFORER UNE CAVITÉ CORPORELLE ET CANULE DESTINÉE À ÊTRE UTILISÉE AVEC UN CATHÉTER QUI PEUT ÊTRE DÉPLACÉ DANS LA CANULE

(30) Priority: 19.10.2011 FR 1103211
(43) Date of publication of application: 27.08.2014
(73) Proprietor: B.Braun Medical SAS, 92210 Saint-Cloud (FR)
(72) Inventor: COLLIN, Rémi, 61354 Saint-Hilaire-sur-Erre (FR)
(74) Representative: August Debouzy
(86) International application number: PCT/EP2012/004383
(87) International publication number: WO 2013/064215

(56) References cited:
- EP-A1- 1 364 675
- EP-A1- 1 437 155
- EP-A1- 1 832 235
- EP-A2- 0 159 033
- WO-A1-2004/035126
- WO-A1-2007/004531
- WO-A2-88/03035
- DE-A1- 3 347 150
- DE-A1- 4 200 595
- FR-A5- 2 129 116
- GB-A- 904 237
- US-A- 3 827 434
- US-A- 4 846 791
- US-A1- 2003 088 212
- US-A1- 2005 027 256
- US-A1- 2006 200 081
- US-B1- 6 887 417

## Description

The present invention concerns a catheter with a removable cannula for puncturing body cavities as well as a cannula of such type.

Several areas of medicine regularly call for laying pathways for accessing body cavities to remain on the patient for prolonged period. To this purpose, the body cavity must be punctured with a cannula before introducing a catheter therein. This is, for example, the case for central vein catheters or for subpubic puncture of the bladder through the abdominal and vesical walls.

In most known processes, the catheter is guided in the cannula. The puncture of the body cavity is conducted in a first step with the cannula. The catheter is then inserted into the body cavity via the cannula before the latter is removed. The cannula is afterwards situated on the catheter and must be removed. Since the end of the catheter opposite the patient is generally provided with means for connection to other devices, the cannula cannot be removed via that end.

Several solutions are known for removing the cannulas from catheters in such cases. According to document DE 2 204 211, a cannula can be provided with a rupture line of the tubular wall along which it can be separated into two parts, then removed from the catheter. Separating the cannula nevertheless requires a lot of strength and may cause the formation of protruding edges and hence the risk of injuries. On the other hand, the catheter may be damaged. Other solutions are described in FR 2 129 116 A5 defining the preamble of the independent claims, GB 904,237 A, US 4,846,791 and EP 1347155 A1.

The aim of the present invention is to provide a catheter with a puncturing cannula which may be removed from the catheter without risk of injury for the patient and the nursing staff nor risk of damaging the equipment. Another object is to provide a catheter with a puncturing cannula whose tip is normally protected or unaccessible so as to exclude any inadvertent risk of injury for the persons handling the cannula and this, before as well as after puncturing the body cavity.

According to the invention, this double object is satisfied by a catheter with removable cannula for puncturing a body cavity, whereas the catheter can be moved freely in the longitudinal direction in the cannula. The cannula is characterised in that it is tubular in shape and provided with a longitudinal slot extending over 90° to 180° of the circumference of the cannula and in that it is provided in a tubular sheath so that they can be displaced in the longitudinal direction inside said tubular sheath. Advantageously, the cannula may be secured in the sheath either in a retracted position where the tip of the cannula is protected by the end of the sheath, or in an extended position where the tip of the cannula protrudes beyond the end of the sheath. When the cannula/sheath assembly is fixed in the extended position, it is ready for puncturing a body cavity.

According to the invention, the cannula exhibits a longitudinal slot which extends over 45° to 180°, preferably slightly less than 180°, of the circumference of the cannula. In practice, it has proven appropriate to choose longitudinal slots extending over 130° to 170° and especially over 150° of the circumference of the cannula. A catheter adapted to the inner diameter of the cannula and inserted therein may thus be removed from the cannula along the longitudinal slot. The cannula is arranged inside a tubular sheath so that it can be moved therein in a controlled manner. It may especially be a pipe made of flexible plastic material. The inner diameter of this tubular sheath is adapted to the external diameter of the cannula. The catheter is then held securely in the cannula in spite of the longitudinal slot.

The object of the invention is also satisfied by a catheter with the features described above which is characterized in that the tubular sheath is arranged in the cannula so that it can be moved in the longitudinal direction inside said cannula, whereas the catheter is arranged inside the tubular sheath. The invention is described below with reference to the embodiment having a tubular sheath outside the cannula. However, the man of the art can obviously also arrange the tubular sheath inside the cannula.

For puncturing a body cavity, for example, the bladder, the cannula is moved in the peripheral tubular sheath so that the tip of the cannula protrudes therefrom so that the puncture can take place. Once the puncture has been done, the catheter is inserted into the body cavity via the cannula. The catheter is thus guided securely into the cannula via the tubular sheath.

After inserting the catheter, the cannula is first brought back into a retracted position with respect to the tubular sheath, then the cannula/tubular sheath assembly is removed. The catheter can be moved freely in the cannula, its end stays in the body cavity whereas the cannula and the tubular sheath are removed to be located on a section of the catheter outside the patient's body.

According to the described embodiment, there are different methods for removing the cannula from the catheter. The first comprises separating the tubular sheath from the cannula. The longitudinal slot in the cannula is then exposed. The cannula can be removed from the catheter. The tubular sheath does not present any risky element and can remain on the catheter, especially when the tubular sheath is made of flexible plastic material.

Optionally, the tubular sheath may be provided with a rupture line in its wall in the longitudinal direction located just above the slot of the cannula. The tubular sheath can be broken or split along the rupture line and thus enable to remove the cannula/tubular sheath assembly from the catheter. The risk of injuries is here particularly low when the tubular sheath is made of flexible plastic.

Alternately, the sheath may also exhibit two rupture lines, parallel to one another, so that a longitudinal section can be removed from its wall. The longitudinal section can be advantageously removed from the wall of the tubular sheath while the tubular sheath is still situated on the cannula. The longitudinal slot in the cannula is thus exposed and the cannula can be removed from the catheter with the rest of the tubular sheath. The tip of the cannula is still protected by the tubular sheath after having been removed from the catheter which enables preventing from any risk of injury.

If the tubular sheath is provided with two rupture lines between which a longitudinal section of the wall can be removed, the width of this section must preferably be adapted to that of the longitudinal slot of the cannula. The wall section between the rupture lines is preferably connected to a manipulation section with which the wall section can be removed along the rupture lines.

In an embodiment, the cannula and the tubular sheath exhibit fittings for connecting both pieces to each other removably. Preferably, both pieces are snapped to each other, when the cannula and the tubular sheath are in a position in which the tip of the cannula is included in the tubular sheath and it hence protected against injuring the nursing staff.

The aim of the present invention is then satisfied by a cannula to be used with a catheter and having the features described above.

According to the application domain, the cannula may exhibit different diameters. The outer diameter of a cannula inserted into a tubular sheath is preferably smaller of the order of 0.1 mm than the inner diameter of the sheath to be able to slide easily inside the sheath.

The invention is explained more accurately below using the appended figures:
- **Figures 1a to 1c**: illustrate a first embodiment of the cannula according to the invention in a perspective view;
- **Figures 2a to 2d**: illustrate a second embodiment of the cannula according to the invention in a perspective view;
- **Figures 3a to 3b**: illustrate a third embodiment of the cannula according to the invention in transverse cross section.

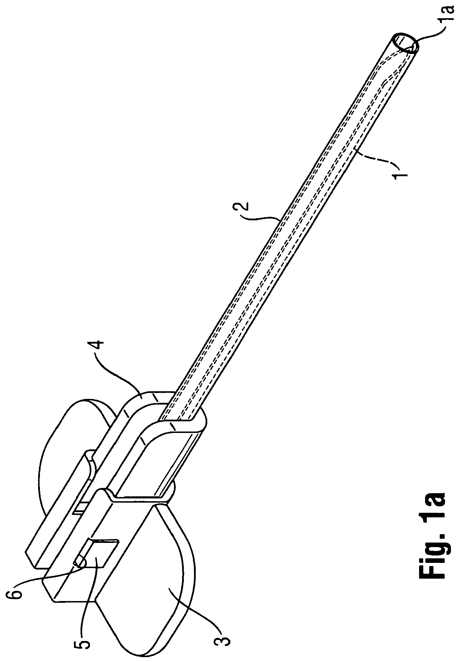

**Figure 1a** represents a cannula **1** according to the invention arranged so that it can be moved in a tubular sheath **2** of flexible plastic. The cannula and the tubular sheath respectively present fittings **3, 4,** connected to one another removably. The cannula **1** has an inner diameter to house a catheter - not represented in the Figures - which can be inserted into the cannula so that it can move along the longitudinal direction. Figure 1a illustrates the cannula **1** as delivered. The length of the tubular sheath **2** is dimensioned so that the tip 1a of the cannula **1** is retracted inside the tubular sheath **2** and does not represent any danger.

A catheter not represented in the figure can be inserted into the cannula from the end of the cannula on which the fittings **3, 4** are arranged. The catheter is first of all inserted until its end stays inside of the cannula **1.**

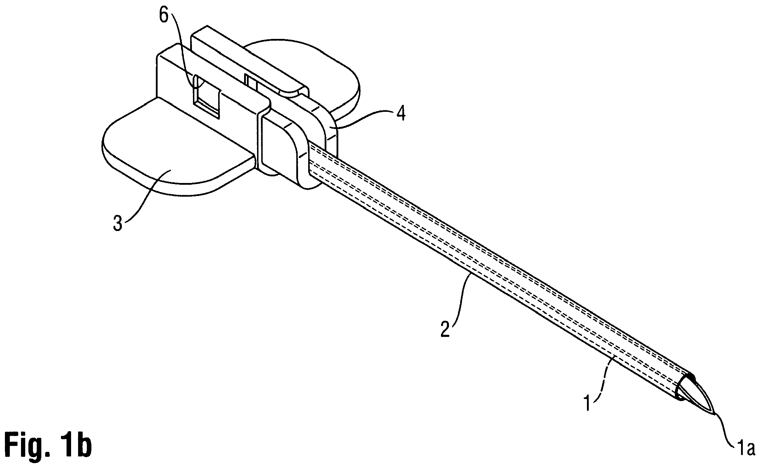

It can be seen on **figure 1b** **that** the fittings of the cannula **1** and of the tubular sheath **2** can be moved relative to one another so that the cannula **1** can be moved within the tubular sheath so that its tip 1a protrudes from the tubular sheath **2.** The cannula **1** may thus be used for puncturing a body cavity.

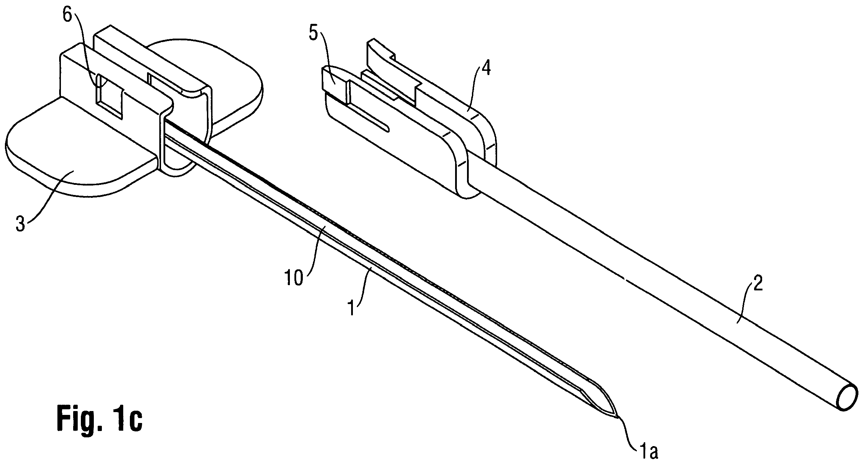

The fittings **3, 4** are designed in the represented embodiment so that they can be guided inside one another in the longitudinal direction. The fitting **4** of the tubular sheath **2** therefore includes notches **5** (fig. 1a) snapping into the hollows **6** on the fittings **4** of the cannula **1** when the latter is completely retracted into the tubular sheath 2 (fig. 1). The snapping position excludes any possibility of unintentionally extending the tip of the cannula **1a** outside the tubular sheath **2** and any risk of injury. For the cannula **1** to go from the delivery condition according to Figure 1a to the puncture condition according to Figure 1b, the notches **5** must be depressed. The tubular sheath **2** can only then be moved backwards.

If the body cavity is punctured with the cannula **1,** the catheter can be inserted into the body cavity via the cannula **1.** The cannula **1** can then be removed along with the tubular sheath **2.** If the cannula **1** and the tubular sheath **2** are completely extracted from the patient's body, they are still on the catheter. The cannula **1** then represents a significant risk of injuries and should be removed. To this purpose, the cannula **1** is extracted from the tubular sheath **2.** The fittings **3, 4** are therefore separate from one another and the fitting **4** of the tubular sheath **2** is pushed in the direction of the tip of the cannula **1.** The cannula 1 can then be removed completely from the tubular sheath **2,** as shown in Figure 1c, The longitudinal slot **10** enables to remove the catheter from the cannula **1.** It is not necessary to break the cannula **1** or to treat it in any other form.

The tubular sheath **2** is still situated on the catheter which is not represented in the figures. It is made of plastic and does not represent any particular risk of injury. It may stay on the catheter or be removed therefrom, e.g. by breaking it along a rupture line not represented. Contrary to the metal cannulas to be broken, protruding edges representing a significant risk of injuries do not risk forming on the rims of the plastic tubular sheath.

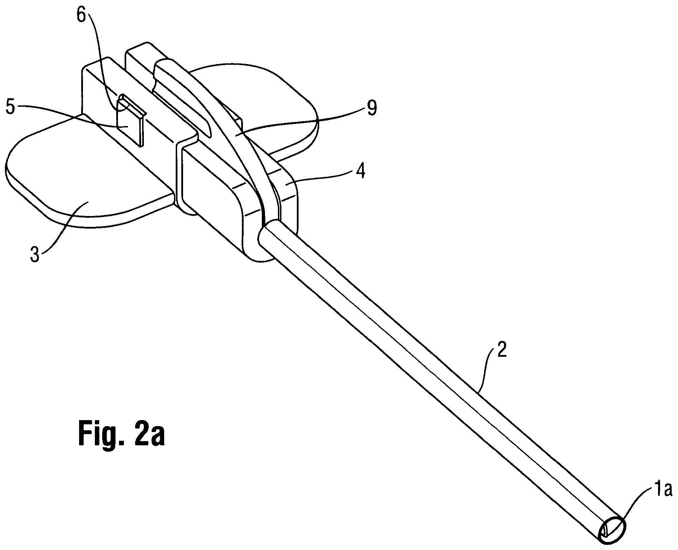

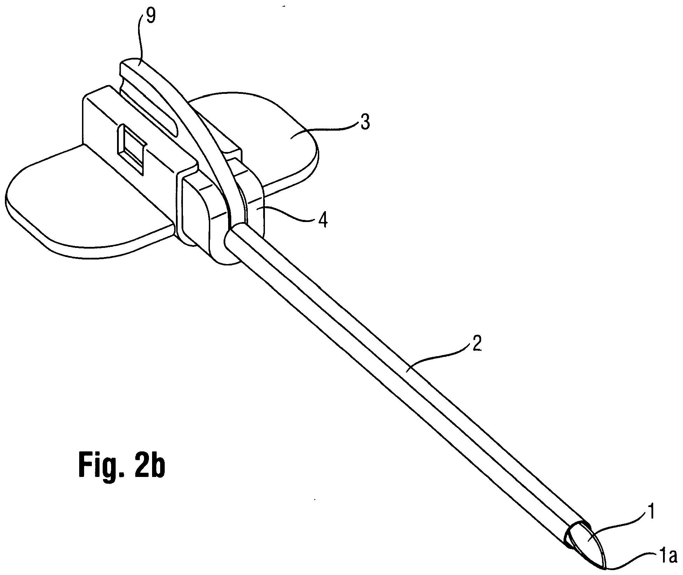

**Figures 2a to 2d** represent a second embodiment of a cannula **1** according to the invention. Figures **2a** **and** **2b** essentially correspond to Figures 1a and 1b. Figure 2a illustrates a cannula **1** as delivered with a tubular sheath **2.** To achieve the condition represented in figure 2b, the fittings **3, 4** are moved relative to one another here as well after snapping the notches **5** so that the tip **1a** of the cannula **1** comes out of the tubular sheath **2** and that the cannula **1** may be used for the puncture.

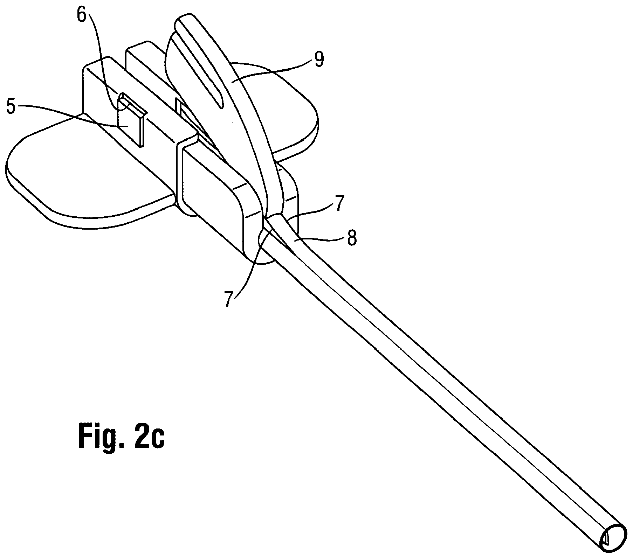

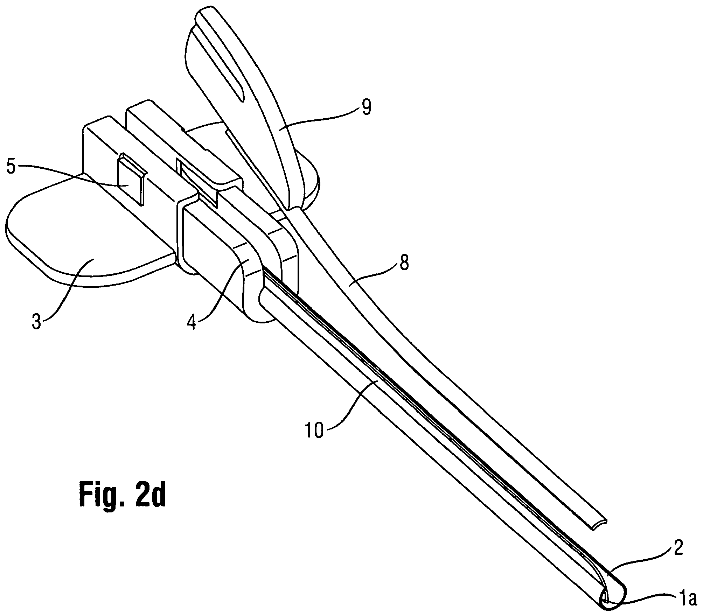

As indicated in **figure 2c****,** the tubular sheath **2** of the embodiment of Figures 2, contrary to the embodiment of Figures 1, is fitted with two rupture lines **7** as well as a manipulation section **9** connected to the wall section **8** between the rupture lines **7.** If the cannula **1** is withdrawn from the patient's body, the tubular sheath is first of all again moved forward in the direction of the tip **1a** of the cannula until the notches **5** snap into the hollow sections **6.** The tip **1a** of the cannula **1** is then brought back into the tubular sheath **2** and protected.

To remove the cannula from the catheter, the wall section **8** of the tubular sheath **2** between the rupture lines **7** is withdrawn using the manipulation section **9.** To this purpose, the manipulation section **9** is pulled upwards as shown on Figure 2c.

It can be seen on **Figure 2d** that the withdrawal of the wall section **8** between the rupture lines **7** clears the longitudinal slot **10** from the cannula **1.** The cannula **1** can thus be removed from the catheter without problems. Fitting the connection elements **3, 4** using notches enables to connect the tubular sheath **2** and the cannula **1** to one another durably, even once the catheter has been withdrawn. The tip **1a** of the catheter **1** is still protected in the remaining part of the tubular sheath and does not present any risk of injury.

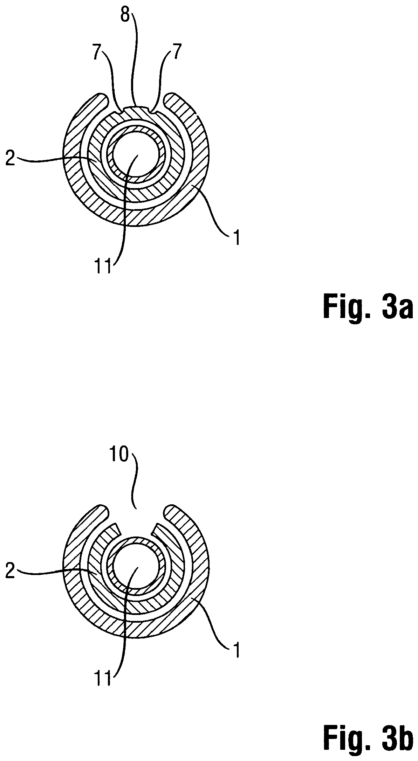

**Figures 3a to 3b** illustrate a third embodiment of the cannula according to the invention in transverse cross section. As indicated in **figure 3a****,** the tubular sheath **2** is arranged in this embodiment inside the cannula **1.** Same as for the other embodiments, the sheath **2** can be moved in the longitudinal direction in the cannula **1.** In a first protection position, the sheath **2** protrudes from the tip of the cannula **1** and thereby excludes the risk of needle jab on the tip. In a second puncture position, the sheath **2** is withdrawn towards the inside of the cannula, and the puncture can be carried out.

The sheath **2** includes a catheter **11** which may be fed forward after puncture for insertion into the body cavity. The cannula **1** is then withdrawn. To be removed from the catheter **11,** the sheath **2** exhibits two rupture lines **7** for removing the wall section **8** therebetween.

**Figure 3b** shows the assembly formed by the cannula **1,** the tubular sheath **2** and the catheter **11** after removing the wall section between the rupture lines **7.** The wall section removed from the sheath **2** corresponds to the slot **10** of the cannula. The cannula **1** and the sheath **2** can be removed from the catheter **11** without any the risk of injury or damaging the catheter **11.**

## Claims

1. A catheter with removable cannula (1) for puncturing a body cavity,
whereas the catheter can be moved in the longitudinal direction in the cannula (1), wherein
the cannula (1) is tubular in shape and provided with a longitudinal slot (10),
whereas said longitudinal slot (10) extends over 45° to 180° of the circumference of the cannula(1),
**characterized in that** the cannula (1) and the catheter are arranged in a tubular sheath (2) so that it can be displaced in the longitudinal direction inside said tubular sheath (2).

2. A catheter with removable cannula (1) for puncturing a body cavity,
whereas the catheter can be moved in the longitudinal direction in the cannula (1) wherein
the cannula (1) is tubular in shape and provided with a longitudinal slot (10),
whereas said longitudinal slot (10) extends over 45° to 180° of the circumference of the cannula (1),
**characterized in that** a tubular sheath (2) is arranged inside the cannula so that it can be displaced in the longitudinal direction inside said cannula (1),
whereas the catheter is arranged inside the tubular sheath.

3. A catheter according to claims 1 or 2, **characterised in that** the longitudinal slot (10) of the cannula (1) extends over 45° to 180° of the circumference of the cannula (1), in particular over 150°.

4. A catheter according to any of the claims 1 to 3, **characterised in that** the tubular sheath (2) is made of plastic, in particular made of flexible plastic.

5. A catheter according to any of the claims 1 to 4, **characterised in that** the tubular sheath (2) exhibits at least one rupture line (7), preferably two, in the longitudinal direction.

6. A catheter according to claim 5, **characterised in that** the tubular sheath (2) exhibits two rupture lines (7) and **in that** the wall section (8) between the rupture lines (7) is connected to a manipulation section (9) and can be removed.

7. A catheter according to any of the claims 1 to 6, **characterised in that** the cannula (1) and the tubular sheath (2) exhibit respectively a fitting element (3, 4) enabling to connect them to one another removably.

8. A catheter according to any of the claims 1 to 7, **characterised in that** the fitting elements (3, 4) of the cannula (1) and of the tubular sheath (2) are snapped to one another in a position in which the tip (1a) of the cannula (1) is included in the tubular sheath (2).

9. A cannula (1) for the use with a catheter which can be moved in the cannula (1) wherein
the cannula (1) is tubular in shape and provided with a longitudinal slot (10),
whereas said longitudinal slot (10) extends over45° to 180° of the circumference of the cannula (1),
**characterized in that** the cannula (1) is arranged inside a tubular sheath (2) so that it can be displaced in the longitudinal direction inside said sheath (2).

10. A cannula (1) for the use with a catheter which can be moved in the cannula (1) wherein
the cannula (1) is tubular in shape and fitted with a longitudinal slot (10),
whereas said longitudinal slot (10) extends over 45° to 180° of the circumference of the cannula (1),
**characterized in that** a tubular sheath (2) is arranged inside the cannula so that it can be displaced in the longitudinal direction inside said cannula (1).

11. A cannula (1) according to claim 8, **characterised in that** the longitudinal slot (10) of the cannula (1) extends over 130° to 170° of the circumference of the cannula (1), in particular 150°.

12. A cannula (1) according to any of the claims 9 to 11, **characterised in that** the tubular sheath (2) is made of plastic, in particular made of flexible plastic.

13. A cannula (1) according to any of the claims 9 to 12, **characterised in that** the tubular sheath (2) exhibits at least one rupture line (7), preferably two, in the longitudinal direction.

14. A cannula (1) according to claim 13, **characterised in that** the tubular sheath (2) exhibits two rupture lines (7) and **in that** the wall section (8) between the rupture lines (7) is connected to a manipulation section (9) and can be removed.

15. A cannula (1) according to any of the claims 9 to 14, **characterised in that** the cannula (1) and the tubular sheath (2) exhibit respectively a fitting element (3, 4) enabling to connect them to one another removably.

16. A cannula (1) according to any of the claims 9 to 15, **characterised in that** the fitting elements (3, 4) of the cannula (1) and of the tubular sheath (2) are snapped to one another in a position in which the tip (1a) of the cannula (1) is enclosed in the tubular sheath (2).

## Patentansprüche

1. Katheter mit abnehmbarer Kanüle (1) zur Punktion einer Körperhöhle,
wobei der Katheter im Inneren der Kanüle (1) in Längsrichtung bewegbar ist,
wobei
die Kanüle (1) rohrförmig und mit einem länglichen Schlitz (10) versehen ist,
wobei sich der längliche Schlitz (10) über 45 - 180 ° des Umfangs der Kanüle (1) erstreckt, **dadurch gekennzeichnet, dass** Kanüle (1) und Katheter in einer rohrförmigen Hülle (2) angeordnet sind, damit die Kanüle und der Katheter im Inneren der rohrförmigen Hülle (2) in Längsrichtung verschoben werden kann.

2. Katheter mit abnehmbarer Kanüle (1) zur Punktion einer Körperhöhle,
wobei der Katheter im Inneren der Kanüle (1) in Längsrichtung bewegbar ist,
wobei
die Kanüle (1) rohrförmig und mit einem länglichen Schlitz (10) versehen ist,
wobei sich der längliche Schlitz (10) über 45 - 180 ° des Umfangs der Kanüle (1) erstreckt, **dadurch gekennzeichnet, dass** im Inneren der Kanüle eine rohrförmige Hülle (2) angeordnet ist, damit diese im Inneren der Kanüle (1) in Längsrichtung verschoben werden kann,
wobei der Katheter im Inneren der rohrförmigen Hülle angeordnet ist.

3. Katheter nach Anspruch 1 oder 2, **dadurch gekennzeichnet, dass** sich der längliche Schlitz (10) der Kanüle (1) über 45 - 180 ° des Umfangs der Kanüle (1), insbesondere über 150 ° erstreckt.

4. Katheter nach einem der Ansprüche 1 - 3, **dadurch gekennzeichnet, dass** die rohrförmige Hülle (2) aus Kunststoff, insbesondere aus flexiblem Kunststoff, besteht.

5. Katheter nach einem der Ansprüche 1 - 4, **dadurch gekennzeichnet, dass** die rohrförmige Hülle (2) mindestens eine Sollbruchleine (7), vorzugsweise zwei, in Längsrichtung aufweist.

6. Katheter nach Anspruch 5, **dadurch gekennzeichnet, dass** die rohrförmige Hülle (2) zwei Sollbruchleinen (7) aufweist und der Wandabschnitt (8) zwischen den Sollbruchleinen (7) mit einem Manipulationsabschnitt (9) verbunden ist und entfernt werden kann.

7. Katheter nach einem der Ansprüche 1 - 6, **dadurch gekennzeichnet, dass** Kanüle (1) und rohrförmige Hülle (2) jeweils ein Passtück (3, 4) aufweisen, das es ihnen ermöglicht, sich trennbar miteinander zu verbinden.

8. Katheter nach einem der Ansprüche 1 - 7, **dadurch gekennzeichnet, dass** die Passstücke (3, 4) von Kanüle (1) und rohrförmiger Hülle (2) miteinander in einer Position zusammengerastet sind, in der die Spitze (1a) der Kanüle (1) in der rohrförmigen Hülle (2) enthalten ist.

9. Kanüle (1) zur Verwendung mit einem in der Kanüle (1) bewegbaren Katheter, wobei die Kanüle (1) rohrförmig und mit einem länglichen Schlitz (10) versehen ist,
wobei sich der längliche Schlitz (10) über 45 - 180 ° des Umfangs der Kanüle (1) erstreckt, **dadurch gekennzeichnet, dass** die Kanüle (1) in einer rohrförmigen Hülle (2) angeordnet ist, damit sie im Inneren der Hülle (2) in Längsrichtung verschoben werden kann.

10. Kanüle (1) zur Verwendung mit einem in der Kanüle (1) bewegbaren Katheter, wobei die Kanüle (1) rohrförmig und mit einem länglichen Schlitz (10) versehen ist,
wobei sich der längliche Schlitz (10) über 45 - 180 ° des Umfangs der Kanüle (1) erstreckt, **dadurch gekennzeichnet, dass** im Inneren der Kanüle eine rohrförmige Hülle (2) angeordnet ist, damit diese im Inneren der Kanüle (1) in Längsrichtung verschoben werden kann.

11. Kanüle (1) nach Anspruch 8, **dadurch gekennzeichnet, dass** sich der längliche Schlitz (10) der Kanüle (1) über 130 - 170° des Umfangs der Kanüle (1), insbesondere über 150 ° erstreckt.

12. Kanüle (1) nach einem der Ansprüche 9 - 11, **dadurch gekennzeichnet, dass** die rohrförmige Hülle (2) aus Kunststoff, insbesondere aus flexiblem Kunststoff, besteht.

13. Kanüle (1) nach einem der Ansprüche 9 - 12, **dadurch gekennzeichnet, dass** die rohrförmige Hülle (2) mindestens eine Sollbruchleine (7), vorzugsweise zwei, in Längsrichtung aufweist.

14. Kanüle (1) nach Anspruch 13, **dadurch gekennzeichnet, dass** die rohrförmige Hülle (2) zwei Sollbruchleinen (7) aufweist und der Wandabschnitt (8) zwischen den Sollbruchleinen (7) mit einem Manipulationsabschnitt (9) verbunden ist und entfernt werden kann.

15. Kanüle nach einem der Ansprüche 9 - 14, **dadurch gekennzeichnet, dass** Kanüle (1) und rohrförmige Hülle (2) jeweils ein Passtück (3, 4) aufweisen, das es ihnen ermöglicht, sich trennbar miteinander zu verbinden.

16. Kanüle (1) nach einem der Ansprüche 9 - 15, **dadurch gekennzeichnet, dass** die Passstücke (3, 4) von Kanüle (1) und rohrförmiger Hülle (2) miteinander in einer Position zusammengerastet sind, in der die Spitze (1a) der Kanüle (1) in der rohrförmigen Hülle (2) enthalten ist.

## Revendications

1. Cathéter avec canule amovible (1) pour ponction d'une cavité corporelle,
le cathéter pouvant être déplacé dans la direction longitudinale dans la canule (1),
dans lequel
la canule (1) est de forme tubulaire et pourvue d'une fente longitudinale (10),
ladite fente longitudinale (10) s'étend sur 45° à 180° de la circonférence de la canule (1), **caractérisé en ce que** la canule (1) et le cathéter sont agencés dans une gaine tubulaire (2) de manière à pouvoir être déplacés dans la direction longitudinale à l'intérieur de ladite gaine tubulaire (2).

2. Cathéter avec canule amovible (1) pour ponction d'une cavité corporelle,
le cathéter pouvant être déplacé dans la direction longitudinale dans la canule (1),
dans lequel
la canule (1) est de forme tubulaire et pourvue d'une fente longitudinale (10),
ladite fente longitudinale (10) s'étend sur 45° à 180° de la circonférence de la canule (1), **caractérisé en ce qu'**une gaine tubulaire (2) est agencée à l'intérieur de la canule de manière à pouvoir être déplacée dans la direction longitudinale à l'intérieur de ladite canule (1),
le cathéter étant agencé à l'intérieur de la gaine tubulaire.

3. Cathéter selon les revendications 1 ou 2, **caractérisé en ce que** la fente longitudinale (10) de la canule (1) s'étend sur 45° à 180° de la circonférence de la canule (1), en particulier sur 150°.

4. Cathéter selon l'une quelconque des revendications 1 à 3, **caractérisé en ce que** la gaine tubulaire (2) est réalisée en plastique, en particulier réalisée en plastique souple.

5. Cathéter selon l'une quelconque des revendications 1 à 4, **caractérisé en ce que** la gaine tubulaire (2) présente au moins une ligne de rupture (7), de préférence deux, dans la direction longitudinale.

6. Cathéter selon la revendication 5, **caractérisé en ce que** la gaine tubulaire (2) présente deux lignes de rupture (7) et **en ce que** la section de paroi (8) entre les lignes de rupture (7) est reliée à une section de manipulation (9) et peut être enlevée.

7. Cathéter selon l'une quelconque des revendications 1 à 6, **caractérisé en ce que** la canule (1) et la gaine tubulaire (2) présentent respectivement un élément de raccord (3, 4) permettant de les raccorder l'une à l'autre, de manière amovible.

8. Cathéter selon l'une quelconque des revendications 1 à 7, **caractérisé en ce que** les éléments de raccord (3, 4) de la canule (1) et de la gaine tubulaire (2) sont encliquetés entre eux dans une position dans laquelle la pointe (1a) de la canule (1) est comprise dans la gaine tubulaire (2).

9. Canule (1) pour l'utilisation avec un cathéter pouvant être déplacé dans une canule (1), dans laquelle
la canule (1) est de forme tubulaire et pourvue d'une fente longitudinale (10),
ladite fente longitudinale (10) s'étend sur 45° à 180° de la circonférence de la canule (1), **caractérisée en ce que** la canule (1) est agencée à l'intérieur d'une gaine tubulaire (2) de manière à pouvoir être déplacée dans la direction longitudinale à l'intérieur de ladite gaine (2).

10. Canule (1) pour l'utilisation avec un cathéter qui peut être déplacé dans une canule (1), dans laquelle
la canule (1) est de forme tubulaire et pourvue d'une fente longitudinale (10),
ladite fente longitudinale (10) s'étend sur 45° à 180° de la circonférence de la canule (1), **caractérisée en ce qu'**une gaine tubulaire (2) est agencée à l'intérieur de la canule de manière à pouvoir à être déplacée dans la direction longitudinale à l'intérieur de ladite canule (1).

11. Canule (1) selon la revendication 8, **caractérisée en ce que** la fente longitudinale (10) de la canule (1) s'étend sur 130° à 170° de la circonférence de la canule (1), en particulier 150°.

12. Canule (1) selon l'une quelconque des revendications 9 à 11, **caractérisée en ce que** la gaine tubulaire (2) est réalisée en plastique, en particulier réalisée en plastique souple.

13. Canule (1) selon l'une quelconque des revendications 9 à 12, **caractérisée en ce que** la gaine tubulaire (2) présente au moins une ligne de rupture (7), de préférence deux, dans la direction longitudinale.

14. Canule (1) selon la revendication 13, **caractérisée en ce que** la gaine tubulaire (2) présente deux lignes de rupture (7) et **en ce que** la section de paroi (8) entre les lignes de rupture (7) est reliée à une section de manipulation (9) et peut être enlevée.

15. Canule (1) selon l'une quelconque des revendications 9 à 14, **caractérisée en ce que** la canule (1) et la gaine tubulaire (2) présentent respectivement un élément de raccord (3, 4) permettant de les raccorder l'une à l'autre de manière amovible.

16. Canule (1) selon l'une quelconque des revendications 9 à 15, **caractérisée en ce que** les éléments de raccord (3, 4) de la canule (1) et de la gaine tubulaire (2) sont encliquetés entre eux dans une position dans laquelle la pointe (1a) de la canule (1) est comprise dans la gaine tubulaire (2).
